# EUROPEAN PATENT SPECIFICATION

(11) **EP 1 477 124 B1**
(45) Date of publication and mention of the grant of the patent: **31.10.2007**
(21) Application number: 04252487.6
(22) Date of filing: 29.04.2004
(51) Int. Cl.: A61B 17/80

(54) **Fixation of fractured bones**
Befestigung von gebrochenen Knochen
Fixation de fractures osseuses

(30) Priority: 12.05.2003 GB 0310860
(43) Date of publication of application: 17.11.2004
(73) Proprietor: C.H. Medical Limited, Exeter, Devon EX2 8WA (GB)
(72) Inventor: Harrod, Christopher George Charles, Exeter EX4 4SW (GB); Young, Phillipe Georges Young, Lympstone Devon EX8 5LW (GB); Pappas, Christos Athanasios, Agrinio 30100 (GR)
(74) Representative: Harrison, Ivor Stanley

(56) References cited:
- WO-A-99/38447
- DE-U- 20 022 673
- GB-A- 2 331 244
- US-A- 6 093 201
- US-A1- 2003 055 429

## Description

This invention relates to the fixation of fractured bones and particularly provides a fixation device constituting a plate comprising a body portion and fastening fingers which, in use, straddle the bone in the region of the fracture and grip the bone when subjected to an external inwards clamping force.

Devices of the type described are known. GB 2331244 discloses an arcuate metallic plate, according to the preamble of claim 1, with projecting fastening fingers formed on two edges, that can be positioned around a fractured bone and held in place by deforming the fingers inwardly to cause inwardly directed anchoring projections located at the ends of the fastening fingers to engage the bone.

WO-A-99/38447 discloses a similar device further comprising an array of inwardly extending protrusions which serve to maintain a gap between the device and the bone, thereby helping to avoid undesirable degradation of the bone tissue.

The use of such prior art devices to fix a fractured bone requires a large clamping force to be applied to the device, in order that the anchoring projections at the ends of the fastening fingers penetrate the bone, thus holding the fractured bone in the position in which it is desired that it should heal. However, such clamping forces can be difficult for the surgeon to generate and could entail further damage to the bone or associated soft tissues. In addition, a portion of the deformation of the device induced by the clamping force is lost due to elastic spring-back, which results in the anchoring projections not always penetrating the bone fully, if at all.

Furthermore, especially for long bones such as the femur, greater strength and support may be required than is provided by the devices described above. GB-A-2331244 discloses one means of meeting these requirements, namely, by further fixing the device to the bone by means of screws which project into the bone.

An alternative and preferable means of gaining increased strength and support from the plate, without the need for screwing it into the bone, is to form the plate from a thicker material or a stronger and/or stiffer material such as cobalt chrome or titanium. However, plates of the general type described above are typically manufactured from a single sheet of metal and therefore forming a plate from thicker material or a stronger and/or stiffer material would result in an even greater clamping force being required in order to cause the anchoring projections to penetrate the bone. In addition, the use of stronger materials such as those suggested above increases the percentage elastic spring-back.

An object of the present invention is to enable the use of thicker or stronger materials and the construction of a stronger stiffer plate, overcoming the problems mentioned above by modifying the fastening fingers, such that the required deformation as a result of the clamping force will take place under less force and will be less susceptible to elastic spring-back.

According to a first aspect of the invention there is provided a fixation device for fractured bone, the device comprising a plate having an elongate body portion with fastening fingers protruding from opposite longitudinal edges thereof, said fastening fingers each including one or more inwardly directed anchoring projections, characterised in that each of at least some of the fastening fingers comprises discrete zones exhibiting different pliability relative to the remainder of the finger.

Preferably the plate will comprise a metal, most preferably an alloy selected from biocompatible implantable grade alloys such as stainless steel, cobalt chrome, titanium and memory metal alloy (nitinol).

Alternatively the plate will comprise a biocompatible polymeric plastics material, preferably a bioresorbable polymer.

Alternatively the plate will comprise a biocompatible composite material. It may also include an external coating.

The anchoring projections are intended in use to engage with and penetrate the bone on application of a clamping force to the fastening fingers and may be disposed at the finger ends distal to the body portion of the plate or at an intermediate location. Respective fingers may be formed with one or more anchoring projections at different locations from those on other fingers, to inhibit any tendency for cracks to propagate along the bone. The anchoring projections may be formed with an elongate or bevelled bone-penetration edge which may be disposed laterally or otherwise in relation to the finger. For example, individual fingers may have lateral projections formed at their distal ends and longitudinal projections formed at intermediate locations, intended in use more effectively to hold fractured bone ends together while the bone heals.

Use of the term "discrete zones" in this specification indicates distinct or identifiable portions of a fastening finger which have different pliability or varying pliability as the result of variations in the depth profile along the length of the finger and/or across the finger and/or in planform. These variations have the result that different clamping forces are required to cause the fastening fingers to grip the bone and also that less elastic spring-back occurs. Discrete zones of increased pliability may be introduced into each fastening finger by reducing its thickness in distinct portions as compared with the thickness of other portions of the finger, for example, by way of one or more grooves formed across the width of the fastening fingers and/or by way of one or more grooves formed along the length of the fastening fingers. Preferably, such grooves may extend along at least part of the length of the finger, more preferably along the whole length of the finger. Such grooves may have varying depths and/or widths. Alternatively, discrete zones of increased pliability may be introduced into each fastening finger by reducing the width of the finger. In a further alternative, discrete zones of increased pliability may be introduced into each fastening finger by forming one or more apertures in the fingers.

Conversely, discrete zones of reduced pliability may be introduced into each fastening finger by way, for example, of increasing the width of the finger.

Where the device comprises a metal, the discrete zones of different pliability may alternatively be introduced into a fastening finger by altering the metallurgical microstructure and hence the physical properties along the length of the finger.

The presence of two or more discrete zones of different pliability has the further beneficial effect that, on application of the clamping force, the fingers have compound bend or yield properties along their length, whereby they can be adapted to yield in a selective manner. For example, the presence of two discrete zones of increased pliability in a finger, one towards the end of the finger distal to the body portion of the plate and one towards the end of the finger proximal to the body portion of the plate, has the result that, on application of the clamping force, the finger yields first at the end proximal to the body portion of the plate until the finger makes contact with the bone. Inwardly protruding anchoring projections located towards the end of the finger proximal to the body portion of the plate are then urged to engage with and penetrate the bone. Further application of the clamping force causes the finger to yield further at the end distal to the body portion of the plate, with the bone acting as a fulcrum, such that anchoring projections at the ends of the finger distal to the body portion of the plate are urged to engage with and penetrate the bone. A discrete zone of reduced pliability between the proximal and distal discrete zones of increased pliability would serve to magnify this effect.

Optionally, on the inner, that is, bone facing, surface of a device according to the invention are formed nodules or protrusions which make contact with the bone and maintain a gap between the body portion of the device and the bone.

A further embodiment of the invention includes additional protrusions extending from the edges of the body portion of the plate and defining channels for receiving cables for the purpose of fixing the device to the bone, in addition to the fixing provided by the fastening fingers. Conveniently, such channels constitute a protrusion on the inner side of the plate such that a gap is maintained between the device and the bone, with the exception of the contact made with the bone via the anchoring projections and the channel protrusions. This has the advantageous effect of helping to avoid undesirable degradation of the bone tissue and blood circulatory system that can occur as the result of restricted access of body fluids to the bone and periosteum.

Embodiments of the invention will now be described by way of example only and with reference to the accompanying Figures 1 - 9, in which:
Figure 1 is a plan view of a prior art fixation device;
Figure 2 is a side view of the fixation device shown in Figure 1;
Figure 3 includes a side fragmentary view (A) and alternative end fragmentary views (B, C) of a fixation device according to a first embodiment of the invention;
Figure 4 includes alternative side fragmentary views (A, B) and a cross-section along the line A-A (C) of a device according to a second embodiment of the invention;
Figure 5 includes alternative side fragmentary views (A, B, C) of a device according to a third embodiment of the invention;
Figure 6 includes alternative side fragmentary views (A, B, C) of a device according to a fourth embodiment of the invention;
Figure 7 is a plan view of one end region of a device according to the invention;
Figure 8 is a plan view of the device as shown in Figure 7, in place on the bone; and
Figure 9 is a cross-section of the device shown in Figure 8 along the line B-B.

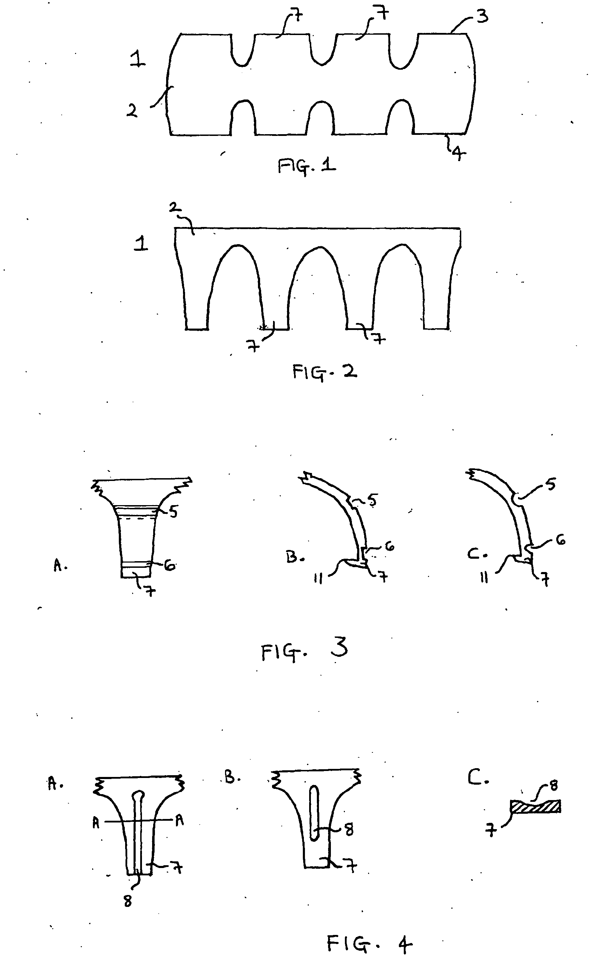

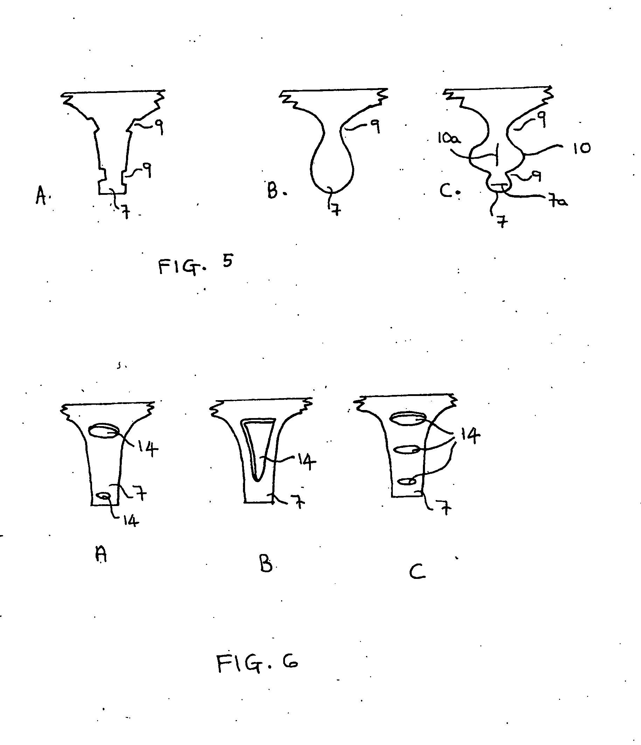

Figures 1 and 2 show a fixation device (1) which has an elongate body portion (2) or spine and an array of fastening fingers (7) along each of its longitudinal edges (3, 4). In embodiments of the device according to the present invention, the fastening fingers are formed in such a way as to have one or more discrete zones of increased pliability or weakness.

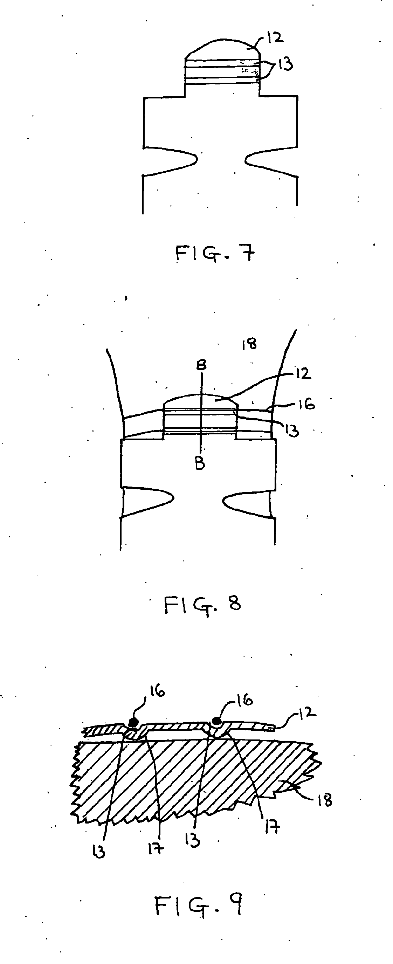

The discrete zones of pliability may be formed by several means. For example, Figure 3 shows a single fastening finger (7) including inwardly directed anchoring projections (11), said fastening finger having two grooves (5, 6) formed across the width of the finger, the grooves being of reduced thickness compared with the rest of the finger. The grooves may have a square cut (Fig. 3A, B) or a rounded (Fig. 3C) profile. Figure 4 shows a single fastening finger (7) which has a groove (8) formed along the length of the finger, the groove being of reduced thickness compared with the rest of the finger (Fig. 4C). The groove may extend along the whole length of the finger (Fig. 4A) or along part of the length of the finger (Fig. 4B). One or more such grooves may be formed in each finger.

An alternative means of forming the discrete zones of pliability is shown in Figure 5. This Figure shows a single fastening finger (7) with regions of reduced width (9) compared with the rest of the finger. Each region of reduced width introduces a discrete zone of increased pliability into the finger. Figure 5C also shows a region of increased width (10) of the finger compared with the rest of the finger, thus introducing a discrete zone of reduced pliability. The locations of elongate anchoring projections, respectively laterally- and longitudinally-disposed, are indicated at 7a and 10a.

A further means of forming the discrete zones of pliability is shown in Figure 6. This Figure shows single fastening fingers (7) with apertures (14) formed in the fingers.

The creation of the zones of pliability by the means described in these embodiments has the result that the device is able to grip the bone as a result of a lesser clamping force than would otherwise be required. Furthermore, the percentage elastic spring-back that may occur once the clamping force ceases is reduced.

In a further embodiment of the invention there are additional protrusions from the edges of the body portion of the device in which are formed channels such that cables may be held in place for the purpose of fixing the device to the bone. Figure 7 shows such a protrusion (12) and the channels (13) formed within it. Figure 8 shows the device attached to the bone (18) by means of cables (16) placed in the channels (13). Figure 9 shows that the channels (13) create protrusions (17) on the inner side of the device which make contact with the bone, thereby maintaining a gap between the main body of the device and the bone. This has the advantageous effect of allowing access of body fluids to the bone and so helping to avoid undesirable degradation of the bone tissue and blood circulatory system.

## Claims

1. A fixation device for fractured bone, comprising a plate having an elongate body portion with fastening fingers protruding from opposite longitudinal edges thereof, said fastening fingers each including one or more inwardly directed anchoring projections, **characterised in that** each of at least some of the fastening fingers comprises discrete zones comprising variations in the thickness of the finger which exhibit different pliability relative to the remainder of the finger, wherein, when the fastening fingers grip the bone, elastic spring-back is reduced.

2. A device according to claim 1 in which the discrete zones comprise one or more grooves formed across the width of the finger.

3. A device according to claims 1 or 2 in which the discrete zones comprise one or more grooves formed along the length of the finger.

4. A device according to claim 3 in which the one or more grooves extend along at least part of the length of the finger.

5. A device according to any of claims 2-4 in which the one or more grooves are formed with variations in depth.

6. A device according to any of claims 1-5 in which the material forming the plate comprises a metal.

7. A device according to claim 6 in which the metal comprises a biocompatible alloy selected from implant grade stainless steel, cobalt chrome, titanium or nitinol alloys.

8. A device according to any of claims 1-5 in which the material forming the plate comprises a biocompatible polymeric plastics material.

9. A device according to claim 8 in which the polymeric plastics material comprises bioresorbable polymer.

10. A device according to any of claims 1-5 in which the material forming the plate comprises a composite material.

11. A device according to any preceding claim in which the device includes an external coating.

12. A device according to any preceding claim in which the discrete zones comprise variations in the width of the finger.

13. A device according to claim 12 in which the discrete zones comprise one or more indentations in the side edges of the finger.

14. A device according to claim 12 in which the variations in the width of the finger are increases in the width of the finger.

15. A device according to any preceding claim in which the discrete zones comprise one or more apertures in the fingers.

16. A device according to any preceding claim including additional protrusions extending from the edges of the body portion of the plate and defining channels for receiving cables for the purpose of fixing the device to the bone, in addition to the fixing provided by the fastening fingers.

17. A device according to claim 16 in which the channels constitute a protrusion on the inner side of the plate such that when the device is clamped onto the bone the protrusion makes contact with the bone.

## Patentansprüche

1. Eine Vorrichtung zur Fixation von gebrochenen Knochen, bestehend aus einer Platte mit einem verlängerten Körperteil mit abstehenden Befestigungsfingern, mit daran entgegengesetzten längs verlaufenden Kanten, wobei die Befestigungsfinger jeweils eine oder mehrere einwärts gerichtete Verankerungsauskragungen umfassen, **dadurch gekennzeichnet, dass** jeder der Befestigungsfinger wenigstens einen abgesonderten Bereich aufweist, der eine Dickenschwankung in dem Finger umfasst, der eine unterschiedliche Biegsamkeit relativ zu dem Rest des Fingers aufweist, und sobald die Befestigungsfinger den Knochen ergreifen, die elastische Rückfederung reduziert ist.

2. Eine Vorrichtung gemäß Anspruch 1, wobei die abgesonderten Bereiche eine oder mehrere Riefen aufweisen, die quer über die Breite des Fingers ausgebildet sind.

3. Eine Vorrichtung gemäß Anspruch 1 oder 2, wobei die abgesonderten Bereiche eine oder mehrere Riefen umfassen, die entlang der Länge des Fingers ausgebildet sind.

4. Eine Vorrichtung gemäß Anspruch 3, wobei sich eine oder mehrere Riefen längsseits ausdehnen und zumindest einen Teil der Länge des Fingers darstellen.

5. Eine Vorrichtung gemäß einem der Ansprüche 2 bis 4, wobei die Tiefen der Riefen, bei einer oder mehreren Riefen, unterschiedlich tief ausgebildet sind.

6. Eine Vorrichtung gemäß einem der Ansprüche 1 bis 5, wobei das Material, das die Platte ausbildet, ein Metall ist

7. Eine Vorrichtung gemäß Anspruch 6, wobei das Metall aus einer biokompatiblen Legierung von ausgesuchtem Implantatgüteedelstahl, Kobaltchrom, Titanium oder Nitinol-Legierungen besteht.

8. Eine Vorrichtung gemäß einem der Ansprüche 1 bis 5, wobei das Material, das die Platte ausbildet, aus einem biokompatiblen polymerischen Plastikmaterial besteht.

9. Eine Vorrichtung gemäß Anspruch 8, wobei das polymerische Plastikmaterial aus einem bioresorbierbaren Polymer besteht.

10. Eine Vorrichtung gemäß einem der Ansprüche 1 bis 5, wobei das Material, das die Platte ausbildet, ein Kompositmaterial ist.

11. Eine Vorrichtung gemäß einem der vorangehenden Ansprüche, wobei die Vorrichtung eine äußere Ummantelung aufweist.

12. Eine Vorrichtung gemäß einem der vorangehenden Ansprüche, wobei die abgesonderten Bereiche in der Breite der Finger Schwankungen aufweisen.

13. Eine Vorrichtung gemäß Anspruch 12, wobei die abgesonderten Bereiche eine oder mehrere Vertiefungen in den Seitenkanten der Finger aufweisen.

14. Eine Vorrichtung gemäß Anspruch 12, wobei die Schwankungen in der Breite der Finger mit der Breite der Finger zunehmen.

15. Eine Vorrichtung gemäß einem der vorangehenden Ansprüche, wobei die abgesonderten Bereiche eine oder mehrere Öffnungen in den Fingern umfassen.

16. Eine Vorrichtung gemäß einem der vorangehenden Ansprüche, umfassend zusätzliche Vorsprünge entlang der Kanten des Körperteiles der Platte, die Kanäle ausbilden und zur Aufnahme von Kabeln dienen, um die Vorrichtung zusätzlich zu dem Fixieren, das durch die Befestigungsfinger vorgesehen ist, an dem Knochen zu befestigen.

17. Eine Vorrichtung gemäß Anspruch 16, wobei die Kanäle einen Vorsprung an der Innenseite der Platte ausbilden, so dass, wenn die Vorrichtung an dem Knochen eingespannt ist, der Vorsprung im Kontakt mit dem Knochen steht.

## Revendications

1. Dispositif de fixation d'un os fracturé, comprenant une plaque ayant une partie de corps allongée munie de doigts de fixation qui dépassent de ses bords longitudinaux opposés, les doigts de fixation comprenant chacun une ou plusieurs saillies d'ancrage dirigées vers l'intérieur, **caractérisé en ce que** chacun d'au moins certains doigts de fixation comporte des zones délimitées comprenant des variations d'épaisseur du doigt, qui présentent une souplesse différente par rapport au reste du doigt, et telles que, lorsque les doigts de fixation serrent l'os, la force de retour élastique est réduite.

2. Dispositif selon la revendication 1, dans lequel les zones délimitées comportent une ou plusieurs gorges formées suivant la largeur du doigt.

3. Dispositif selon la revendication 1 ou 2, dans lequel les zones délimitées comprennent une ou plusieurs gorges formées suivant la longueur du doigt.

4. Dispositif selon la revendication 3, dans lequel la gorge ou les gorges d'étendent suivant une partie au moins de la longueur du doigt.

5. Dispositif selon l'une quelconque des revendications 2 à 4, dans lequel la gorge ou les gorges sont formées avec des variations de profondeur.

6. Dispositif selon l'une quelconque des revendications 1 à 5, dans lequel le matériau formant la plaque est un métal.

7. Dispositif selon la revendication 6, dans lequel le métal est un alliage biocompatible choisi parmi l'acier inoxydable, le chrome au cobalt, des alliages de titane et le "Nitinol", de qualité pour implant.

8. Dispositif selon l'une quelconque des revendications 1 à 5, dans lequel le matériau formant la plaque est une matière plastique polymère biocompatible.

9. Dispositif selon la revendication 8, dans lequel la matière plastique polymère est un polymère biorésorbable.

10. Dispositif selon l'une quelconque des revendications 1 à 5, dans lequel le matériau formant la plaque est un matériau composite.

11. Dispositif selon l'une quelconque des revendications précédentes, dans lequel le dispositif comporte un revêtement externe.

12. Dispositif selon l'une quelconque des revendications précédentes, dans lequel les zones délimitées comportent des variations de largeur du doigt.

13. Dispositif selon la revendication 12, dans lequel les zones délimitées comportent un ou plusieurs évidements aux bords latéraux du doigt.

14. Dispositif selon la revendication 12, dans lequel les variations de largeur du doigt sont des augmentations de largeur du doigt.

15. Dispositif selon l'une quelconque des revendications précédentes, dans lequel les zones délimitées comportent un ou plusieurs orifices dans les doigts.

16. Dispositif selon l'une quelconque des revendications précédentes, comprenant des saillies supplémentaires qui s'étendent depuis les bords de la partie de corps de la plaque et qui délimitent des canaux pour le logement de câbles destinés à la fixation du dispositif à l'os, en plus de la fixation assurée par les doigts de fixation.

17. Dispositif selon la revendication 16, dans lequel les canaux constituent une saillie du côté interne de la plaque si bien que, lorsque le dispositif est serré sur l'os, la saillie est au contact de l'os.
